# EUROPEAN PATENT APPLICATION

(11) **EP 4 162 894 A1**
(43) Date of publication of application: **12.04.2023**
(21) Application number: 21821057.3
(22) Date of filing: 04.06.2021
(51) Int. Cl.: A61B 34/37, A61B 34/30

(54) **ATTITUDE ADJUSTING MECHANISM, MAIN MANIPULATOR DEVICE AND SURGICAL ROBOT**

(30) Priority: 08.06.2020 CN 202010512332
(71) Applicant: Suzhou Kangduo Robot Co., Ltd., Suzhou, Jiangsu 215153 (CN)
(72) Inventor: WANG, Jianguo, Suzhou, Jiangsu 215153 (CN); ZHANG, Jiaxing, Suzhou, Jiangsu 215153 (CN)
(74) Representative: Zaboliene, Reda
(86) International application number: PCT/CN2021/098282
(87) International publication number: WO 2021/249293

(57) **Abstract**

The present invention provides an attitude adjusting mechanism, a main manipulator device, and a surgical robot, relating to the technical field of medical instruments. The attitude adjusting mechanism includes: a first link, a third flange, a second link, and a main hand clamp, one end of the second link being hinged with one end of the first link so as to rotate the first link about a second axial direction; the other end of the second link is rotatably connected to the third flange so as to enable the second link to rotate around the third axial direction, and the main hand clamp is hinged to the other end of the first link so as to enable the main hand clamp to rotate around the first axial direction; the second axial direction is perpendicular to the third axial direction, the third axial direction is perpendicular to the first axial direction, and the second axial direction is perpendicular to the first axial direction.

## Description

### Technical Field

The present invention relates to the technical field of medical instruments, and more particularly, to an attitude adjusting mechanism, a main manipulator device, and a surgical robot.

### Background Art

In medical operations, such as surgical operations, the fine operation objects are small in size and complex in shape, which often takes a long time, making the doctor easily tired. Therefore, human hand tremors and fatigue easily lead to inaccuracies in surgical actions, and a master-slave controlled mechanical hand enables a doctor to perform surgical operations in a relatively comfortable attitude so that a master-slave controlled surgical system has received much attention in the industry. However, the structural design of the manipulator of an existing surgical robot is unreasonable, and especially the structural design of the attitude portion of the main manipulator is unreasonable, which leads to the inconvenience in the surgical procedure and especially can not meet the needs of complex surgeries.

### Summary of the Invention

The present invention is intended to solve, to some extent, the problem that, however, the structural design of the manipulator of an existing surgical robot is unreasonable, especially the structural design of the attitude portion of the main manipulator is unreasonable, which leads to the inconvenience in the surgical procedure and especially can not meet the needs of complex surgeries.

In order to solve the above problem, the present invention provides an attitude adjusting mechanism comprising:
a first link;
a third flange;
a second link having one end hinged to one end of the first link to rotate the first link about a second axial direction;
wherein the other end of the second link is rotatably connected to the third flange such that the second link is adapted to rotate around a third axial direction;
and the main hand clamp hinged to the other end of the first link to rotate the main hand clamp about a first axial direction;
wherein the second axial direction is perpendicular to the third axial direction, the third axial direction is perpendicular to the first axial direction, and the second axial direction is perpendicular to the first axial direction.

Further, the other end of the first link is provided with a groove body, the groove body penetrating the first link in a thickness direction of the first link, wherein the main hand clamp is hinged at the groove body via a first connecting shaft, and an axis of the first connecting shaft is the first axial direction.

Further, a balancing weight is provided at a position of the first connecting shaft away from the main hand clamp.

Further, the first connecting shaft is rotatably connected to the groove body via a first rotating shaft and a bearing, one end of the second link is rotatably connected to one end of the first link via a second rotating shaft and a bearing, and the other end of the second link is rotatably connected to the third flange via a third rotating shaft and a bearing.

Further, a second detection mechanism is provided at a hinge joint of the first link and the second link for detecting an angle of rotation of the first link around the second axial direction;
a third detection mechanism is provided at a connection of the second link and the third flange for detecting the angle of rotation of the second link around the third axial direction;
a first detection mechanism is provided at the hinge joint of the main hand clamp and the first link for detecting the angle of rotation of the main hand clamp around the first axial direction.

Further, the first detection mechanism is a first motor for controlling the main hand clamp to rotate around the first axial direction, the second detection mechanism is a second motor for controlling the first link to rotate around the second axial direction, and the third detection mechanism is a third motor for controlling the first link to rotate around the second axial direction, wherein an encoder is respectively integrated inside the first motor, the second motor, and the third motor;
or the other end of the first link is provided with a groove body, the groove body penetrating through the first link along the thickness direction of the first link, wherein the main hand clamp is hinged at the groove body via the first connecting shaft, and the axis of the first connecting shaft is the first axial direction; the first connecting shaft is rotatably connected to the groove body via a first rotating shaft and a bearing, one end of the second link is rotatably connected to one end of the first link via a second rotating shaft and a bearing, and the other end of the second link is rotatably connected to the third flange via a third rotating shaft and a bearing, wherein one end of the first rotating shaft, the second rotating shaft, and the third rotating shaft is respectively connected with one encoder.

In addition, the present invention also provides a main manipulator device including the attitude adjusting mechanism;
and a position adjusting mechanism (2) comprising:
a straight line mechanism, wherein a sliding block is provided at the straight line mechanism, the sliding block is adapted to move along the length direction of the straight line mechanism, and a connecting base is provided at the sliding block;
a connecting rod, one end of the connecting rod being connected to the third flange, the other end of the connecting rod being connected to the connecting base, and the sliding block being adapted to move along a fourth axial direction;
a sliding support, one end of the straight line mechanism being hinged to the sliding support so that the straight line mechanism is adapted to rotate around a fifth axial direction;
and a base, the sliding support being hinged to the base so that the sliding support is adapted to rotate around a sixth axial direction;
wherein the fourth axial direction is perpendicular to and non-intersecting with the fifth axial direction, the fifth axial direction is perpendicular to the sixth axial direction, and the fourth axial direction is perpendicular to the sixth axial direction.

Further, a fifth motor is provided at the sliding support, the fifth motor being adapted to control the straight line mechanism to rotate around the fifth axial direction, and a sixth motor is provided at the base, the sixth motor being adapted to control the sliding support to rotate around the sixth axial direction.

In addition, the present invention provides a surgical robot including the main manipulator device.

Since the technical improvement of the surgical robot and the obtained technical effect are the same as those of the main manipulator device, the technical effect of the surgical robot will not be described in detail.

Further, the surgical robot is an orthopedic surgical robot or a dental surgical robot, or a laparoscopic surgical robot.

Compared with the prior art, the present invention provides an attitude adjusting mechanism, which has the following technical effects, but is not limited thereto:

The rotation of the main hand clamp about the first axial direction is referred to as a pitching motion, the rotation of the main hand clamp about the third axial direction is referred to as a yaw motion, and the rotation of the main hand clamp about the second axial direction is referred to as a rotary motion; the pitching motion, the yaw motion, and the rotary motion respectively correspond to the pitching motion, the yaw motion, and the rotary motion of the thumb and the index finger in the human hand operation such that the three-degree-of-freedom surgical operation for the main hand clamp is implemented, enabling more complex surgical operations, such as laparoscopic surgery; therefore, the problem that the structural design of the manipulator of an existing surgical robot is unreasonable, especially the structural design of the attitude portion of the main manipulator is unreasonable, which leads to the inconvenience in the surgical procedure and especially can not meet the needs of complex surgeries is solved.

### Brief Description of the Drawings

Fig. 1 is a schematic structural diagram of a main manipulator device according to a specific implementation mode of the present invention;
Fig. 2 is a partially schematic structural diagram of a main manipulator device according to a specific implementation mode of the present invention;
Fig. 3 is a schematic top view of a straight line mechanism of a main manipulator device of a specific implementation mode of the present invention.

### Description of reference numerals:

1-attitude adjusting mechanism, 11-first link, 111-second cylinder, 112-groove body, 12-first connecting shaft, 121-balancing weight, 13-main hand clamp, 131-clamp, 14-third flange, 15-second link, 16-first motor, 17-second motor, and 18-third motor;
2-position adjusting mechanism, 21-straight line mechanism, 211-long plate, 212-rail groove, 213-fourth motor, 214-lead screw, 22-sliding block, 23-connecting base, 24-connecting rod, 25-sliding support, 251-U-shaped groove, 26-base, 27-fifth motor, and 28-sixth motor.

### Detailed Description of the Invention

To make the above objects, features, and advantages of the present invention more apparent, a detailed description of specific embodiments of the present invention will be made with reference to the accompanying drawings.

In the description of the present invention, it is to be understood that the orientation or positional relationships indicated by the terms "up", "down", "front", "rear", etc. are based on the orientation or positional relationship shown in the drawings, and are only for the convenience of describing the invention and simplifying the description, rather than indicating or implying that the referred device or element must have a specific orientation, or be constructed and operated in a specific orientation, and therefore cannot be construed as a limitation of the present invention.

Furthermore, in the drawings, the Z axis represents vertical direction, i.e. up and down positions, and the positive direction of the Z axis (i.e. the pointing of the arrow of the Z axis) represents up, and the negative direction of the Z axis (i.e. the direction opposite to the positive direction of the Z axis) represents down; in the drawings, the X axis represents the longitudinal direction of the horizontal plane, and is perpendicular to the Z axis, and the positive direction of the X axis (i.e., the pointing of the arrow of the X axis) represents the front side, and the negative direction of the X axis (i.e., the direction opposite to the positive direction of the X axis) represents the rear side; in the drawings, Y represents the transverse direction of the horizontal plane, and is perpendicular to the Z axis and the X axis at the same time, and the positive direction of the Y axis (i.e., the pointing of the arrow of the Y axis) represents the left side, and the negative direction of the Y axis (i.e., the direction opposite to the positive direction of the Y axis) represents the right side; the plane formed by the X axis and the Z axis is a vertical plane.

Meanwhile, it needs to be noted that the foregoing Z axis, Y axis, and X axis representations are merely intended to facilitate and simplify the description of the present invention, and are not intended to indicate or imply that the device or element being referred to must have a particular orientation, be constructed and operated in a particular orientation, and thus should not be construed as limiting the invention.

The appearance of the terms "first", "second"... "sixth" are for descriptive purposes only and are not to be construed as indicating or implying relative importance or implicitly indicating the number of technical features indicated. Therefore, a feature defined as "first", "second" ..."sixth" may explicitly or implicitly include at least one such feature.

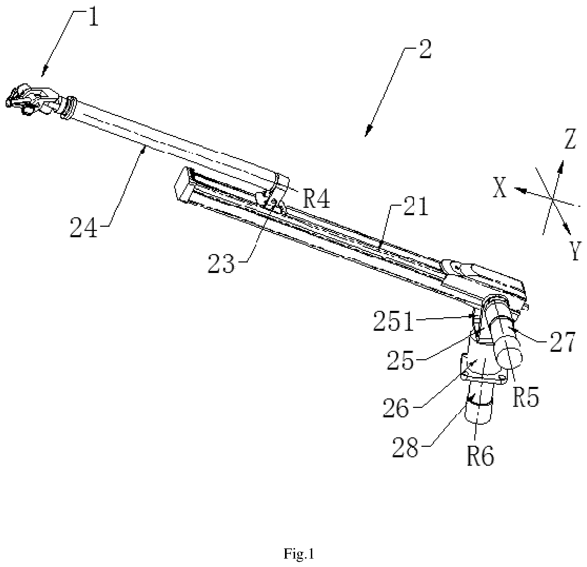

Referring to Figs. 1 to 3, the present implementation mode provides a main manipulator device including an attitude adjusting mechanism 1 and a position adjusting mechanism 2 connected to the attitude adjusting mechanism 1. The attitude adjusting mechanism 1 includes: a first link 11, a third flange 14, and a main hand clamp 13; one end of a second link 15 is hinged with one end of the first link 11 to rotate the first link 11 about a second axial direction; the other end of the second link 15 is rotatably connected to the third flange 14 so that the second link 15 is adapted to rotate around a third axial direction; the main hand clamp 13 is hinged to the other end of the first link 11 to rotate the main hand clamp 13 about a first axial direction.

The first link 11 and the second link 15 can be a straight-rod structure, the second axial direction is perpendicular to the third axial direction, the third axial direction is perpendicular to the first axial direction, and the second axial direction is perpendicular to the first axial direction. For example, the first axial direction may be perpendicular to and non-intersecting with the second axial direction, the second axial direction may be perpendicular to and intersecting with the third axial direction, and the first axial direction may be perpendicular to and intersecting with the third axial direction.

It needs to be noted that the attitude adjusting mechanism 1 in this implementation mode may also be referred to as an attitude portion, and the position adjusting mechanism 2 may also be referred to as a position portion.

Here, the rotation of the main hand clamp 13 about the first axial direction is referred to as a pitching motion, the rotation of the main hand clamp 13 about the third axial direction is referred to as a yaw motion, and the rotation of the main hand clamp 13 about the second axial direction is referred to as a rotary motion; the pitching motion, the yaw motion, and the rotary motion provided via the attitude portion for the main hand clamp 13 respectively correspond to the pitching motion, the yaw motion, and the rotary motion of the thumb and the index finger in the human hand operation, and the pitching motion, the yaw motion, and the rotary motion of the attitude portion realize a three-degree-of-freedom of the surgical operation, which can complete more complicated surgical operations, such as laparoscopic surgery.

In addition, two clamps 131 of the main hand clamp 13 itself are adapted to perform a folding or unfolding motion, which is referred to as a folding and unfolding motion of the main hand clamp 13 itself, corresponding to the folding and unfolding of a thumb and an index finger in a human hand operation, and making it more flexible and changeable.

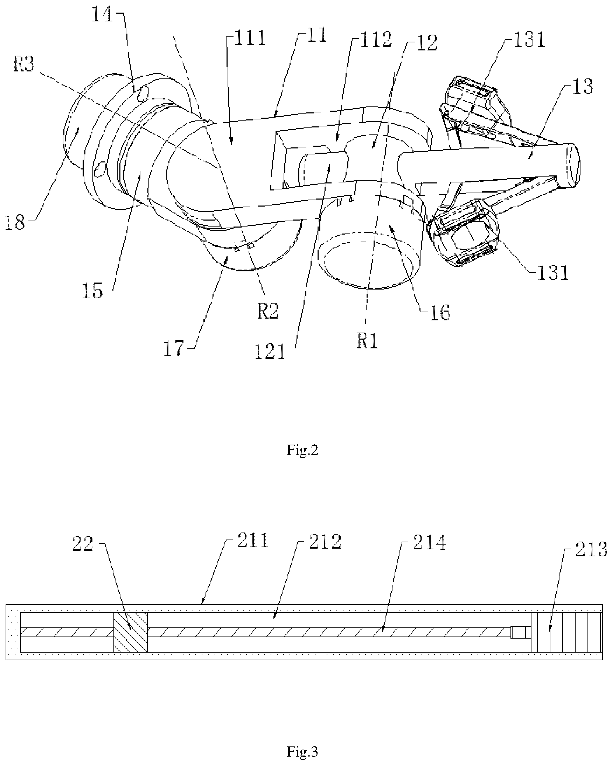

Referring to Fig. 2, preferably, one end of the first link 11 can be a disc-shaped structure, and is denoted as a second cylinder 111, and the other end of the first link 11 is provided with a groove body 112; the groove body 112 penetrates through the first link 11 along the thickness direction of the first link 11, wherein the main hand clamp 13 is hinged at the groove body 112 via the first connecting shaft 12, and the axis R1 of the first connecting shaft 12 is in a first axial direction; the axis R3 of the third flange 14 is in a third axial direction, and one end of the second link 15 is rotatably connected to the second cylinder 111, and the axis R2 of the second cylinder 111 is in a second axial direction.

By providing a groove body 112 at the other end of the first link 11 and hinging the main hand clamp 13 in the groove body 112, the space occupied by the whole attitude portion can be reduced and the structure can be compact.

Referring to Figs. 1 to 3, it is preferable that the position of the first connecting shaft 12 away from the main hand clamp 13 is provided with a balancing weight 121 adapted to adjust the balance of gravity when the main hand clamp 13 rotates about the axis R1 of the first connecting shaft 12.

Here, by providing the balancing weight 121 on one side of the first connecting shaft 12 opposite to the main hand clamp 13, the material of the balancing weight 121 is preferably a material with a high density, so as to ensure that the mass of the balancing weight 121 with a small volume is similar to the mass of the main hand clamp 13, thereby achieving the gravity balance of the main hand clamp 13 when performing a pitching motion. In the meantime, the balancing weight 121 with a small volume does not occupy too much space, so that the whole attitude portion does not occupy too much space.

Referring to Figs. 1 to 3, a detailed description will be made herein of how the first connecting shaft 12 is rotatably connected to the groove body 112, how the first link 11 is rotatably connected to the second link 15, and how the second link 15 is rotatably connected to the third flange 14. Preferably, the first connecting shaft 12 is rotatably connected at the groove body 112 by means of a first rotating shaft and a bearing, wherein the axis of the first rotating shaft is coaxial with the axis R1 of the first connecting shaft 12. One end of the second link 15 is rotatably connected at the second cylinder 111 by means of a second rotating shaft and a bearing, wherein the axis of the second rotating shaft is coaxial with the axis R2 of the second cylinder 111. The other end of the second link 15 is rotatably connected at the third flange 14 by means of a third rotating shaft and a bearing, wherein the axis of the third rotating shaft is coaxial with the axis R3 of the third flange 14.

It is easy to understand that the axis of the first rotating shaft is the axis R1 of the first connecting shaft 12, the axis of the second rotating shaft is the axis R2 of the second cylinder 111, and the axis of the third rotating shaft is the axis R3 of the third flange 14.

Referring to Figs. 1 to 3, preferably, a second detection mechanism for detecting the angle of rotation of the first link 11 about the second axial direction is provided at the hinge joint of the first link 11 and the second link 15; the connection between the second link 15 and the third flange 14 is provided with a third detection mechanism for detecting the rotation angle of the second link 15 about a third axial direction; a first detection mechanism is provided at the hinge joint of the main hand clamp 13 and the first link 11 for detecting the rotation angle of the main hand clamp 13 about the first axial direction.

By providing multiple detection mechanisms, the accurate control of the pitching motion, yaw motion, and rotary motion of the attitude portion is facilitated.

For example, the detection mechanism may be an encoder; a second detection mechanism provided at the hinge joint of the first link 11 and the second link 15 is denoted as a second encoder; a third detection mechanism provided at the connection of the second link 15 and the third flange 14 is a third encoder; a first detection mechanism provided at the hinge joint of the main hand clamp 13 and the first link 11 is denoted as a first encoder.

With reference to Figs. 1 to 3, the connecting relationship of each encoder is specifically described herein; preferably, the first encoder is provided at one end of the first connecting shaft 12, a rotor of the first encoder is fixed with the first rotating shaft, a stator of the first encoder is fixed with the first link 11, the second encoder is provided at one end of the second link 15 connected with the second cylinder 111, the rotor of the second encoder is fixed with the second rotating shaft, the stator of the second encoder is fixed with the second link 15, and a third encoder is provided at one end of the second link 15 connected with the third flange 14; the rotor of the third encoder is fixed to the third rotating shaft and the stator of the third encoder is fixed to the third flange 14.

The first encoder, the second encoder, and the third encoder respectively collect the rotational motion parameter of the main hand clamp 13 when performing the pitching motion, the rotational motion parameter of the main hand clamp 13 when performing the yaw motion, and the rotational motion parameter of the main hand clamp 13 when performing the rotary motion, so as to facilitate the accurate control of the rotation motion of each degree of freedom of the attitude portion.

It needs to be noted that in addition to providing the above-mentioned first encoder, second encoder, and third encoder, it is also possible to replace the first encoder, the second encoder, and the third encoder with a first motor 16, a second motor 17, and a third motor 18, respectively. The first motor 16, the second motor 17, and the third motor 18 are all motors with an encoder integrated therein, namely, the active motions of three degrees of freedom of the attitude portion are achieved by the first motor 16, the second motor 17, and the third motor 18.

Referring to Figs. 1 to 3, preferably, the axis R1 of the first connecting shaft 12 is perpendicular to and non-intersecting with the axis R2 of the second cylinder 111, the axis R2 of the second cylinder 111 is perpendicular to and intersecting with the axis R3 of the third flange 14, and the axis R1 of the first connecting shaft 12 is perpendicular to and intersecting with the axis R3 of the third flange 14.

By the spatial arrangement that "the axis R1 of the first connecting shaft 12 is perpendicular to and non-intersecting with the axis R2 of the second cylinder 111, the axis R2 of the second cylinder 111 is perpendicular to and intersecting with the axis R3 of the third flange 14, and the axis R1 of the first connecting shaft 12 is perpendicular to and intersecting with the axis R3 of the third flange 14", the assembly of the attitude portion can be completed in a small space, and the distribution of the degrees of freedom of the attitude portion makes the operating space of the structure not limited by the interference of the structure motion itself so that the space utilization rate is high. At the same time, this arrangement of degrees of freedom of the attitude section completely matches the arrangement of degrees of freedom of the end instrument of the laparoscopic surgical robot.

Referring to Fig. 1, the position adjusting mechanism 2 includes: a straight line mechanism 21, a connecting rod 24, a sliding support 25, and a base 26.

A sliding block 22 is provided at the straight line mechanism 21, the sliding block 22 is adapted to move along the length direction of the straight line mechanism 21, and a connecting base 23 is provided at the sliding block 22; one end of the connecting rod 24 is connected to the third flange 14, the other end of the connecting rod 24 is connected to the connecting base 23, and the sliding block 22 is adapted to move along the fourth axial direction; one end of the straight line mechanism 21 is hinged with the sliding support 25 so that the straight line mechanism 21 is adapted to rotate around the fifth axial direction; the sliding support 25 is hinged to the base 26 such that the sliding support 25 is adapted to rotate around the sixth axial direction.

The fourth axial direction is perpendicular to and non-intersecting with the fifth axial direction, the fifth axial direction is perpendicular to the sixth axial direction, and the fourth axial direction is perpendicular to the sixth axial direction. For example, the fifth axial direction may be perpendicular to and intersecting with the sixth axial direction, and the fourth axial direction may be perpendicular to and intersecting with the sixth axial direction.

Referring to Fig. 1, preferably, the sliding support 25 is U-shaped, one end of the straight line mechanism 21 is connected to the U-shaped groove 251 of the sliding support 25 via a fifth rotating shaft and a bearing, and the straight line mechanism 21 is adapted to rotate around the axis R5 of the fifth rotating shaft; the space occupied by the main manipulator device can be further reduced.

The base 26 and the sliding support 25 are connected to the base 26 via a sixth rotating shaft and a bearing, the sliding support 25 being adapted to rotate around the axis R6 of the sixth rotating shaft.

It could be understood that the axis R4 of the connecting rod 24 is in the fourth axial direction, the axis R5 of the fifth rotating shaft is in the fifth axial direction, and the axis R6 of the sixth rotating shaft is in the sixth axial direction.

Here, the position portion provides motions of three other degrees of freedom by the following modes: "one end of the connecting rod 24 is connected with the third flange 14, and the other end of the connecting rod 24 is connected with the connecting base 23, so as to realize the connection between the position portion and the attitude portion, wherein the moving direction of the sliding block 22 is located on the axis R4 of the connecting rod 24", "the straight line mechanism 21 is connected to the U-shaped groove 251 of the sliding support 25 through the fifth rotating shaft and the bearing, and the straight line mechanism 21 is adapted to rotate around the axis R5 of the fifth rotating shaft", and "the sliding support 25 is connected with the base 26 through the sixth rotating shaft and the bearing, and the sliding support 25 is adapted to rotate around the axis R6 of the sixth rotating shaft".

It needs to be noted that the interior of the connecting rod 24 is hollow and the third encoder or the third motor 18 at the third flange 14 is located inside the connecting rod 24.

Referring to Fig. 1, it is preferable that an outer position of the sliding support 25 is provided with a fifth motor 27, and the motor shaft of the fifth motor 27 is fixedly connected with the fifth rotating shaft.

The fifth motor 27 drives the straight line mechanism 21 to rotate along the axis R5 of the fifth rotating shaft, and then drives the main hand clamp 13 to rotate along the axis R5 of the fifth rotating shaft, so as to realize the active motion in this degree of freedom. Preferably, the fifth motor 27 is a motor with an encoder integrated therein, so as to facilitate the acquisition of the rotational motion parameter in this degree of freedom.

Referring to Fig. 1, preferably, a sixth motor 28 is provided at the bottom of the base 26, and a motor shaft of the sixth motor 28 is fixedly connected to a sixth rotating shaft.

The sixth motor 28 drives the sliding support 25 to rotate along the axis R6 of the sixth rotating shaft, and then drives the main hand clamp 13 to rotate along the axis R6 of the sixth rotating shaft, so as to realize the active motion in this degree of freedom. Preferably, the sixth motor 28 is a motor with an encoder integrated therein, so as to facilitate the acquisition of the rotational motion parameter in this degree of freedom.

Referring to Fig. 3, the detailed structure of the straight line mechanism 21 will be described. Preferably, the straight line mechanism 21 comprises a long plate 211, wherein the long plate 211 is provided with a rail groove 212 along the length direction thereof, one end of the rail groove 212 is provided with a fourth motor 213, a motor shaft of the fourth motor 213 is provided with a lead screw 214, and the lead screw 214 is rotatably connected in the rail groove 212. The sliding block 22 is provided with a threaded hole therein, the sliding block 22 is threaded on the surface of the lead screw 214 via the threaded hole, and the sliding block 22 is slidably connected in the rail groove 212, the connecting base 23 being located outside the rail groove 212.

Preferably, the fourth motor 213 is a motor with an encoder integrated therein, so as to precisely control the rotation of the motor shaft of the fourth motor 213; the fourth motor 213 drives the lead screw 214 to rotate; the sliding block 22 does not rotate under the limit of the rail groove 212, and only moves along the axial direction of the connecting rod 24, and thus drives the main hand clamp 13 to move along the axis R4 of the connecting rod 24.

The present implementation mode also provides a surgical robot that is a laparoscopic surgical robot. Of course, it is not limited to a laparoscopic surgical robot, but may further be an orthopedic surgical robot, or a dental surgical robot, etc.

By the spatial arrangement that "the axis R4 of the connecting rod 24 is perpendicular to and does not intersect with the axis R5 of the fifth rotating shaft, the axis R5 of the fifth rotating shaft is perpendicular to and intersects with the axis R6 of the sixth rotating shaft, and the axis R4 of the connecting rod 24 is perpendicular to and intersects with the axis R6 of the sixth rotating shaft", the assembly of the position portion can be completed in a small space, and the distribution of the degrees of freedom of the position portion makes the operating space of the structure not limited by the interference of the structure motion itself, and the space utilization rate is high. At the same time, the distribution of each degree of freedom in the position portion in combination with the arrangement of each degree of freedom of the attitude portion completely matches the arrangement of the degree of freedom of the end instrument of the laparoscopic surgical robot, so that the main manipulator of the master-slave operating robot provided by the present invention is more accurate in active and driven correspondence when applied to the laparoscopic surgical robot system.

It needs to be noted that the terms "provided on" and "provided at" as used herein may refer to a variety of connections, such as a fixed connection, a removable connection, etc.

Although the present invention is disclosed as above, the scope of the disclosure of the present invention is not limited thereto. Various changes and modifications may be effected by one skilled in the art without departing from the spirit and scope of the disclosure of the present invention, and it is intended that such changes and modifications fall within the scope of the present invention.

## Claims

1. An attitude adjusting mechanism comprising:
a first link (11);
a third flange (14);
a second link (15) having one end hinged to one end of the first link (14) to rotate the first link (11) about a second axial direction;
wherein the other end of the second link (15) is rotatably connected to the third flange (14) such that the second link (15) is adapted to rotate around a third axial direction;
and a main hand clamp (13) hinged to the other end of the first link (11) to rotate the main hand clamp (13) about a first axial direction;
wherein the second axial direction is perpendicular to the third axial direction, the third axial direction is perpendicular to the first axial direction, and the second axial direction is perpendicular to the first axial direction.

2. The attitude adjusting mechanism according to claim 1, wherein the other end of the first link (11) is provided with a groove body (112), the groove body (112) penetrating the first link (11) in a thickness direction of the first link (11), wherein the main hand clamp is hinged at the groove body (112) via a first connecting shaft (12), and an axis of the first connecting shaft (112) is the first axial direction.

3. The attitude adjusting mechanism according to claim 2, wherein a balancing weight (121) is provided at a position of the first connecting shaft (12) away from the main hand clamp (13).

4. The attitude adjusting mechanism according to claim 2, wherein the first connecting shaft (12) is rotatably connected to the groove body (112) via a first rotating shaft and a bearing, one end of the second link (15) is rotatably connected to one end of the first link (11) via a second rotating shaft and a bearing, and the other end of the second link (15) is rotatably connected to the third flange (14) via a third rotating shaft and a bearing.

5. The attitude adjusting mechanism according to any one of claims 1 to 4, wherein:
a second detection mechanism is provided at a hinge joint of the first link (11) and the second link (15) for detecting an angle of rotation of the first link (11) around the second axial direction;
a third detection mechanism is provided at a connection of the second link (15) and the third flange (14) for detecting the angle of rotation of the second link (15) around the third axial direction;
a first detection mechanism is provided at the hinge joint of the main hand clamp (13) and the first link (11) for detecting the angle of rotation of the main hand clamp (13) around the first axial direction.

6. The attitude adjusting mechanism according to claim 5, wherein the first detection mechanism is a first motor (16) for controlling the main hand clamp (13) to rotate around the first axial direction, the second detection mechanism is a second motor (17) for controlling the first link (11) to rotate around the second axial direction, and the third detection mechanism is a third motor (18) for controlling the first link (11) to rotate around the second axial direction, wherein an encoder is respectively integrated inside the first motor (16), the second motor (17), and the third motor (18);
or the other end of the first link (11) is provided with a groove body (112), the groove body (112) penetrating through the first link (11) along the thickness direction of the first link (11), wherein the main hand clamp (13) is hinged at the groove body (112) via the first connecting shaft (12), and the axis of the first connecting shaft (112) is the first axial direction; the first connecting shaft (12) is rotatably connected to the groove body (112) via a first rotating shaft and a bearing, one end of the second link (15) is rotatably connected to one end of the first link (11) via a second rotating shaft and a bearing, and the other end of the second link (15) is rotatably connected to the third flange (14) via a third rotating shaft and a bearing, wherein one end of the first rotating shaft, the second rotating shaft, and the third rotating shaft is respectively connected with one encoder.

7. A main manipulator device comprising the attitude adjusting mechanism (1) according to any one of claims 1 to 6;
and a position adjusting mechanism (2) comprising:
a straight line mechanism (21), wherein a sliding block (22) is provided at the straight line mechanism (21), the sliding block (22) is adapted to move along the length direction of the straight line mechanism (21), and a connecting base (23) is provided at the sliding block (22);
a connecting rod (24), one end of the connecting rod (24) being connected to the third flange (14), the other end of the connecting rod (24) being connected to the connecting base (23), and the sliding block (22) being adapted to move along a fourth axial direction;
a sliding support (25), one end of the straight line mechanism (21) being hinged to the sliding support (25) so that the straight line mechanism (21) is adapted to rotate around a fifth axial direction;
and a base (26), the sliding support (25) being hinged to the base (26) so that the sliding support (25) is adapted to rotate around a sixth axial direction;
wherein the fourth axial direction is perpendicular to and non-intersecting with the fifth axial direction, the fifth axial direction is perpendicular to the sixth axial direction, and the fourth axial direction is perpendicular to the sixth axial direction.

8. The main manipulator device according to claim 7, wherein a fifth motor (27) is provided at the sliding support (25), the fifth motor (27) being adapted to control the straight line mechanism (21) to rotate around the fifth axial direction, and a sixth motor (28) is provided at the base (26), the sixth motor (28) being adapted to control the sliding support (25) to rotate around the sixth axial direction.

9. A surgical robot, wherein the surgical robot comprises the main manipulator device according to claim 7 or 8.

10. The surgical robot according to claim 9, wherein the surgical robot is an orthopedic surgical robot or a dental surgical robot, or a laparoscopic surgical robot.
